# EUROPÄISCHE PATENTSCHRIFT

(11) **EP 2 918 249 B1**
(45) Veröffentlichungstag und Bekanntmachung des Hinweises auf die Patenterteilung: **29.04.2020**
(21) Anmeldenummer: 15158051.1
(22) Anmeldetag: 06.03.2015
(51) Int. Cl.: A61F 2/24

(54) **SUPRACLAVICULÄRES KATHETERSYSTEM FÜR EINEN TRANSSEPTALEN ZUGANG ZUM LINKEN VORHOF UND LINKEN VENTRIKEL**
SUPRACLAVICULAR CATHETER SYSTEM FOR TRANSSEPTAL ACCESS TO THE LEFT ATRIUM AND LEFT VENTRICLE
SYSTÈME DE CATHÉTER SUPRA-CLAVICULAIRE POUR UN ACCÈS TRANSSEPTAL À L'OREILLETTE GAUCHE ET VENTRICULE GAUCHE

(30) Priorität: 14.03.2014 EP 14160061; 14.03.2014 EP 14160065; 31.07.2014 WO PCT/EP2014/066537
(43) Veröffentlichungstag der Anmeldung: 16.09.2015
(73) Patentinhaber: Venus MedTech (HangZhou), Inc., Hangzhou, Zhejiang (CN)
(72) Erfinder: Götz, Wolfgang, 93051 Regensburg (DE)
(74) Vertreter: Zenz Patentanwälte Partnerschaft mbB

(56) Entgegenhaltungen:
- WO-A1-00/72909
- WO-A1-96/17644
- WO-A1-97/21462
- WO-A1-2011/101128
- WO-A1-2012/099979
- WO-A1-2013/059743
- WO-A2-2011/109813
- WO-A2-2012/177942
- WO-A2-2013/114214

## Beschreibung

Die vorliegende Erfindung betrifft einen medizinischen Positionierkatheter gemäß dem Oberbegriff des Anspruchs 1.

Die vorliegende Erfindung betrifft Katheter zum Ersatz nativer Mitralklappen durch künstliche Mitralklappen, hierin gleichbedeutend auch als Mitralklappenimplantate oder Mitralklappenprothesen bezeichnet.

Während man in der Vergangenheit Aortenklappenimplantate ausschließlich chirurgisch implantiert hat, hat die kathetergestützte Aortenklappenimplantation über die letzten Jahre zunehmend großen Erfolg erfahren. Mittlerweile werden allein in Deutschland derzeit jährlich rund 10.000 Aortenklappenprothesen unter Einsatz eines Katheters implantiert, auf dem das Implantat lösbar befestigt zum Implantationtionsort transportiert wird. Die Implantation mittels Katheter hat dabei zahlreiche, dem Fachmann bekannte Vorteile.

Der Zugang für den Katheter erfolgt zumeist über die Femoralarterie. Da die Femoralarterie nur einen mäßig großen Durchmesser hat, dürfen die verwendeten Katheter nicht dicker als ca. 28 Fr. ("French") sein. Ist die Femoralarterie erkrankt oder zu dünn, so kann alternativ der transapikale Zugang über die Herzspitze gewählt werden.

Die auf dem Katheter zusammengefalteten Klappenprothesen haben derzeit einen Durchmesser von ca. 21 Fr.

Seit geraumer Zeit bestehen nicht zuletzt bei der Anmelderin der vorliegenden Anmeldung Bestrebungen, ein Herzklappenimplantat für den kathetergestützten Ersatz der nativen Mitralklappe zu entwickeln. Neben den erheblich größeren Herausforderungen bei der Entwicklung einer Mitralklappenprothese, gemessen an der zwischenzeitlich abgeschlossenen Entwicklung einsetzbarer Aortenklappenprothesen, aufgrund der ungleich schwierigeren Verankerung und Abdichtung der Mitralklappenprothese stellt auch das Einführen der gefalteten Mitralklappenprothese eine große Herausforderung dar.

Wie für die Aortenklappenprothese gibt es zwei Möglichkeiten für einen Zugang zum Implantationsort. Hierbei handelt es sich wiederum um die Herzspitze ("transapikal") und die Leistengefäße ("transfemoral").

So kann auch die Mitralklappenprothese über die Herzspitze eingeführt werden. Der Zugang zur Herzspitze ist leicht herzustellen, ein bis zu 40 Fr. starker Katheter kann ohne Schwierigkeiten eingeführt werden. Da die Klappenprothese hierbei jedoch über die Herzkammer ("Ventrikel") in den nativen Klappenapparat eingeführt wird, können die dort zahlreich vorliegenden Sehnenfäden die korrekte Positionierung der Herzklappe erschweren.

Alternativ kann die Mitralklappenprothese über die Leistenvene, die Vena femoralis, eingeführt werden. Die Vena femoralis hat einen ausreichenden Durchmesser, der es ermöglicht, auch größere Katheter, wie im Fall der Mitralklappenprothese notwendig, einzuführen. Der Mitralklappen-Katheter muss über die Vena cava superior zur Vorhofscheidewand, dem interatrialen Septum, des Herzens geführt werden. Dort wird die Vorhofscheidewand mittels Punktion geöffnet und der Katheter vom rechten Vorhof in den linken Vorhof geführt. Im linken Vorhof muss die Spitze des Katheters, bezogen auf andere Abschnitte des Katheters, eine Kurve um mehr als 270° durchführen, damit die Spitze des Katheters zusammen mit der hierauf lösbar befestigten, zusammengefalteten Mitralklappenprothese in den nativen Mitralklappenapparat eingeführt werden kann. Derartige transvenöse, transseptale Implantationen einer Mitralklappenprothese scheiterten in erster Linie an dem o. g. überstumpfen oder steilen Winkel im linken Vorhof, welchen der Katheter vollziehen muss. Weder die bisher entwickelten Katheter noch die vergleichsweise großen, gefalteten Klappenprothesen erlauben den notwendigen Winkel im linken Vorhof zu vollziehen.

Die vorgenannten technischen Schwierigkeiten hinsichtlich Katheter und/oder Mitralklappenprothese, wenn auf dem Katheter für die Implantation über die venöse Gefäßseite vorgesehen, werden bis zu ihrer derzeit nicht in Aussicht stehenden Lösung dazu führen, dass Mitralklappenprothesen bis auf weiteres neben dem transapikalen Zugang auch über einen weiteren Zugang implantiert werden müssen, welcher eine Thorakoskopie erfordert. In den mittels Thorakoskopie eröffneten Brustkorb wird das Mitralklappenimplantat über den linken Vorhof direkt in den nativen Mitralklappenapparat eingeführt. Hierzu ist jedoch eine Thorakoskopie mit Eröffnen des Brustkorbes, dem Kollabieren der Lunge und damit eine mechanische Beatmung notwendig. Die Vorteile, die eine Implantation mittels Katheter bieten kann, sind hiermit natürlich nicht zu erzielen. Die Druckschrift WO 2011/109813 A2 offenbart ein Verfahren, eine Vorrichtung und ein System zum gezielten Heranführen einer künstlichen Herzklappe an eine defekte Herzklappe, wobei dazu ebenfalls ein medizinischer Positionierkatheter verwendet wird. Der in der WO 2011/109813 A2 offenbarte Positionierkatheter ist jedoch nicht für den Einsatz bei einer nativen Mitralklappe geeignet.

Die Aufgabe der vorliegenden Erfindung ist, eine Vorrichtung vorzuschlagen, mittels welcher auch eine Mitralklappenprothese mittels Katheter implantiert werden kann.

Die erfindungsgemäße Aufgabe kann durch einen Positionierkatheter mit den Merkmalen des Anspruchs 1 gelöst werden.

Der erfindungsgemäße medizinischer Positionierkatheter dient zur Verwendung beim Implantieren eines Mitralklappenimplantats oder zum Reparieren eines Herzdefekts oder einer Mitralklappe.

Der Positionierkatheter weist einen distalen Endbereich, welcher ein distales Ende des Positionierkatheters umfasst, und einen proximalen Endbereich, welcher ein proximales Ende des Positionierkatheters umfasst, auf.

Der Positionierkatheter weist ferner einen hohl ausgestalteten Führungsabschnitt auf. Der Führungsabschnitt weist einen geraden Abschnitt und wenigstens einen ersten Krümmungsabschnitt und einen zweiten Krümmungsabschnitt auf oder besteht hieraus.

Der erste Krümmungsabschnitt und der zweite Krümmungsabschnitt liegen im distalen Endbereich. Alternativ bilden der erste Krümmungsabschnitt oder der zweite Krümmungsabschnitt das distale Ende des Positionierkatheters aus. Sowohl der erste Krümmungsabschnitt als auch der zweite Krümmungsabschnitt sind distal des geraden Abschnitts angeordnet.

Der erste Krümmungsabschnitt bewirkt eine Biegung oder Krümmung des Führungsabschnitts um 10 bis 120°, bevorzugt um 90 bis 120°.

Erfindungsgemäß bewirkt der zweite Krümmungsabschnitt eine Biegung oder Krümmung des Führungsabschnitts um 20 bis 40° bewirkt und zwischen dem ersten Krümmungsabschnitt und dem zweiten Krümmungsabschnitt ist ein 2 - 6 cm langer Abschnitt (308) angeordnet.

Die erfindungsgemäße Ausgestaltung des distalen Endbereichs des medizinischen Positionierkatheters ermöglicht es, den erfindungsgemäßen Positionierkatheter beim Implantieren eines Mitralklappenimplantats oder zum Reparieren eines Herzdefekts oder einer Mitralklappe zu verwenden. Aus dem Stand der Technik bekannte Positionierkatheter können dazu aufgrund abweichender Gestaltung des distalen Endbereichs nicht verwendet werden.

Wenn hierin von einem "Venenwinkel" die Rede ist, so kann hiermit jener Bereich verstanden werden, in welchem sich die Vena subclavia und Vena jugularis interna zur Vena brachiocephalica vereinen. Wird der Venenwinkel punktiert, so kann hierunter beispielhaft ein Punktieren eines beliebigen der vorgeannten Gefäße im Bereich der Vereinigung verstanden werden.

Wenn hierin von paarweise (sinistra, dextra) vorhandenen Gefäßen die Rede ist, so ist im Zweifel davon auszugehen, dass hierin die "dextra"-Form gemeint ist.

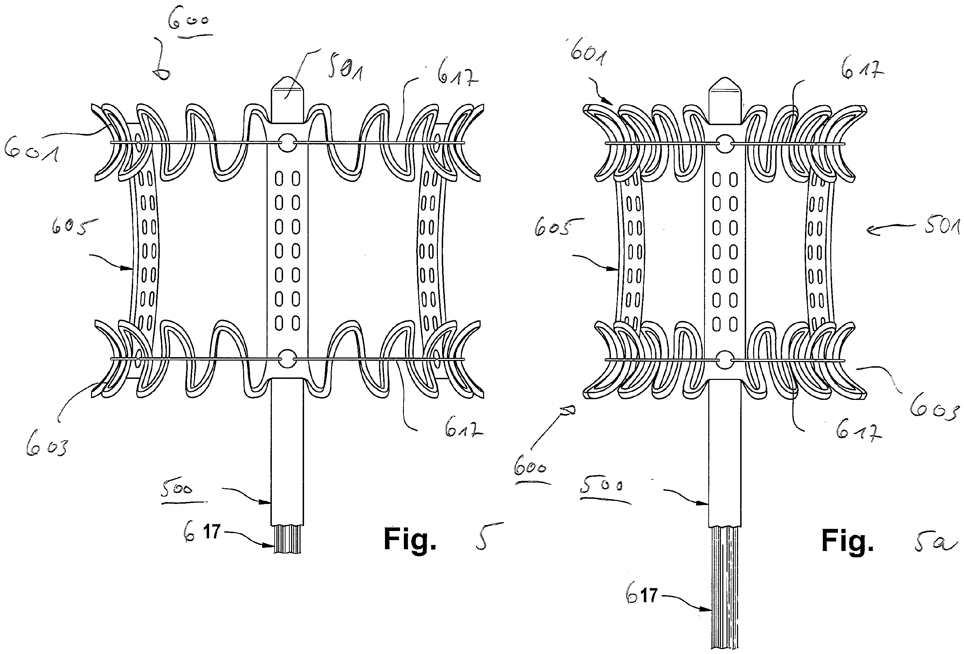

Die Begriffe "distal" und "proximal" beziehen sich hierin auf die Verwendung des betreffenden Katheters. In den hier beigefügten Figuren, mit Ausnahme der Fig. 5 und der Fig. 5a , liegt das proximale Ende eines jeden Katheters im Zweifel immer im oberen Bereich der Figur, während das distale Ende im Zweifel immer im unteren Bereich der Figur liegt. In Fig. 5 und Fig. 5a ist dies umgekehrt.

Erfindungsgemäße Ausführungsformen können eines oder mehrere der im Folgenden genannten Merkmale in beliebiger Kombination aufweisen, sofern eine konkrete Kombination für den Fachmann nicht als offenkundig technisch unmöglich erkennbar ist. Auch die Gegenstände der Unteransprüche geben jeweils erfindungsgemäße Ausführungsformen an.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

In einigen erfindungsgemäßen, beispielhaften Ausführungsformen weist der Positionierkatheter ein Ventil auf, das den geraden Abschnitt nach proximal abschließt.

In manchen erfindungsgemäßen, beispielhaften Ausführungsformen sind der erste Krümmungsabschnitt und der zweite Krümmungsabschnitt des Positionierkatheters gegensinnig gekrümmt, insbesondere bezogen auf eine Ebene. "Gegensinnig" kann sich dabei auf das Vorzeichen der Krümmung (plus, minus) oder des Krümmungswinkels beziehen. "Gegensinnig" kann, beispielsweise bei bildlicher Darstellung der Ebene, in welcher die Krümmungen liegen, als konvex versus konkav oder als "nach links" versus "nach rechts" zu verstehen sein.

In einigen erfindungsgemäßen, beispielhaften Ausführungsformen ist sind der Positionierkatheter oder sein Führungsabschnitt maximal 80 cm, bevorzugt maximal 60 cm, besonders bevorzugt maximal 45 cm lang.

In manchen erfindungsgemäßen, beispielhaften Ausführungsformen sind der Positionierkatheter oder sein Führungsabschnitt nicht-flexibel, nicht-elastisch oder steif.

In einigen erfindungsgemäßen, beispielhaften Ausführungsformen haben der Positionierkatheter, sein Führungsabschnitt, der erste Krümmungsabschnitt und/oder der zweite Krümmungsabschnitt eine shape-memory-Funktion oder - form, bestehen aus einem shape-memory-Material oder weisen ein solches auf.

In manchen erfindungsgemäßen, beispielhaften Ausführungsformen weist der Positionierkatheter ferner ein Ventil auf, das den geraden Abschnitt nach proximal abschließt.

In einigen erfindungsgemäßen, beispielhaften Ausführungsformen trägt der Positionierkatheter in seinem distalen Endbereich lösbar ein Mitralklappenimplantat. Er hat hierzu einen Aufnahmeabschnitt zum Aufnehmen des Mitralklappenimplantats.

In manchen erfindungsgemäßen, beispielhaften Ausführungsformen verläuft die Durchströmungsrichtung des Mitralklappenimplantats, wenn dieses auf dem Positionierkatheter vorliegt, vom proximalen Endbereich zum distalen Endbereich des Positionskatheters.

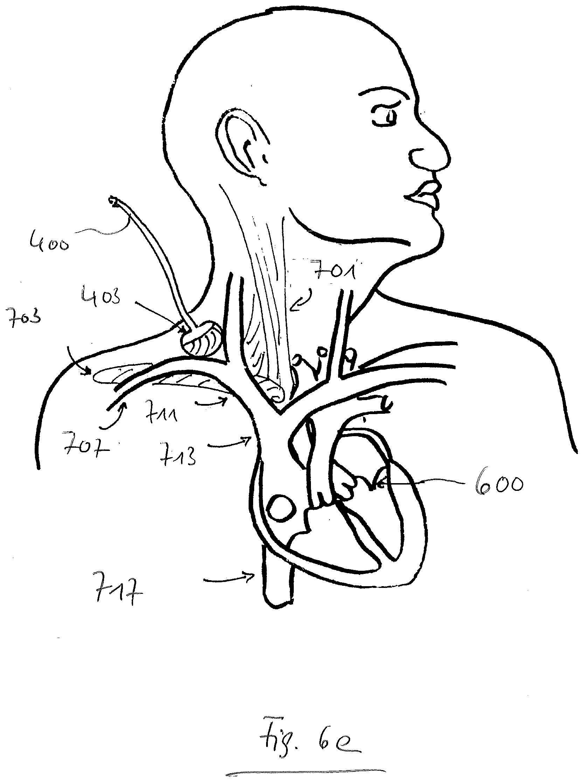

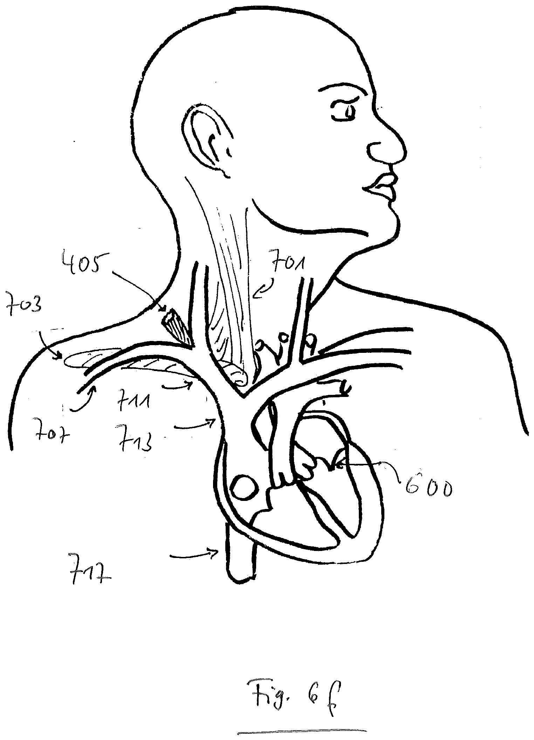

Wenn hierin von der Durchströmungsrichtung des Mitralklappenimplantats die Rede ist, so kann diese als die Richtung verstanden werden, in welcher die Klappen des Implantats nach Implantation einen Durchfluss erlauben, was sie im implantierten Zustand in der hierzu entgegen gesetzten Richtung nicht erlauben würden oder sollten. Die Durchströmungsrichtung haftet dem Implantat unveränderlich daher an, gleich ob es implantiert oder noch auf dem Katheter vorliegt, gleich ob es tatsächlich von Blut durchströmt wird, oder noch nicht. So verläuft beispielsweise die Durchströmungsrichtung des Mitralklappenimplantats 600 der Fig. 6e und 6f bezogen auf die Figur jeweils von oben nach unten. Die Durchströmungsrichtung ist dabei eine strukturelle Eigenschaft des Implantats im Sinne einer Richtung, in welcher das Implantat durchströmbar ist.

In manchen erfindungsgemäßen, beispielhaften Ausführungsformen verläuft die Durchströmungsrichtung des Mitralklappenimplantats, wenn dieses auf dem Mitralklappen-Katheter vorliegt, vom proximalen Endbereich zum distalen Endbereich des Mitralklappen-Katheters.

Der Positionierkatheter ist in bestimmten erfindungsgemäßen Ausführungsformen zwecks - angesichts seines eher steifen Charakters - optimaler Handhabbarkeit nicht über 80 cm lang (gemessen von seinem proximalen Ende bis zu seinem distalen Ende), vorzugsweise zwischen 45 und 60 cm lang.

Der Positionierkatheter kann sterilisiert sein, insbesondere mittels Strahlung, mittels Dampf und/oder mittels Temperatur.

Die Sterilisation hat dabei vorzugsweise werkseitig stattgefunden. Dies erlaubt eine kostengünstige und automatisierte Sterilisation, die wenig für menschliche Fehler anfällig ist.

Der Positionierkatheter kann aus Kunststoff, Polymer, Polyäthylen, Polypropylen, Polyvinylchlorid (PVC), oder Polyamid sein.

Der Positionierkatheter kann einen expandierbaren inneren Durchmesser aufweisen. Dies ist möglich durch eine Elastizität des Rohres oder durch ein Auffalten des Rohres. Entsprechende Ausgestaltungen und Einrichtungen können vorgesehen sein. Somit kann ein Positionierkatheter mit dünnem Durchmesser eingeführt werden. Wird das Mitralklappenimplantat mit einem größeren Durchmesser über den flexiblen Positionierkatheter vorgeschoben, so dehnt sich der Positionierkatheter nur über eine kurze Strecke, nämlich dort, wo das Implantat gerade liegt, und nur so lange, wie es vorgeschoben wird. Dadurch wird das umgebende Gewebe weniger belastet. Noch wichtiger ist, dass der Blutfluss in der Vena brachiocephalika und der Vena cava superior bei liegendem Positionierkatheter vorteilhaft nicht beeinflusst wird. Nur während der kurzen Zeit des Vorschiebens des dickeren Implantates und während sich der flexible Positionierkatheter entsprechend aufdehnt wird der Blutfluss in den erwähnten Venen behindert. Sobald das Implantat den Katheter passiert hat, besteht keine weitere Belastung des Gewebes (Einstichstelle oder Vorhofscheidewand) mehr und der Blutfluss ist nicht mehr behindert.

Der hiermit vorgeschlagene erfindungsgemäße Positionierkatheter erlaubt erstmals eine Mitralklappenimplantation, welche weder transfemoral noch transapikal erfolgt. Dabei können aufgrund der Ausgestaltung des erfindungsgemäßen Positionierkatheters einige der eingangs genannten Schwierigkeiten bei der Implantation aufgehoben oder doch zumindest abgemildert werden.

So erlaubt der erfindungsgemäße Positionierkatheter erstmals eine Implantation über einen supraclaviculären Zugang. Der supraclaviculäre Zugang kann mehrere wesentliche Vorteile bieten. Zu ihnen zählt, dass es im Vergleich zur subclaviculären Punktion klare Orientierungspunkte für die supraclaviculäre Punktion gibt. Die Punktion erfolgt aus der Richtung des Kopfes des Patienten und nicht von der Seite.

Zu ihnen zählt ferner, dass die Entfernung von der Hautoberfläche zur Vene kürzer und die punktierte Vena brachiocephalica hier erheblich dicker ist und sich besser für das Einbringen großer Katheter eignet. Möglich wird dieser Weg jedoch erst durch die spezielle Form der hiermit vorgeschlagenen Katheter.

Zu ihnen zählt ferner, dass ein Katheter zum Einsatz kommen kann, der jedenfalls bis er den rechten Vorhof des Herzens erreicht, gerade verlaufen darf. Erst zum Punktieren des interatrialen Septums muss er vergleichsweise geringfügig gekrümmt sein.

Zu den Vorteilen zählt ferner, dass die Entfernung zur Lunge größer ist als etwa bei dem subclaviculären Zugang, was das Risiko einer Verletzung der Lunge vermindert.

Ein Führungskatheter, welcher zur Vorbereitung der Punktion des interatrialen Septums dient, erlaubt eine einfache, schnelle Punktion des interatrialen Septums nach geradem, supraklavikulärem Zugang.

Der supraclaviculäre Zugang hat gerade für die Implantation einer kathetergestützten Klappenprothese erhebliche Vorteile, die einen perkutane, d.h. mittels Gefäßpunktion durchgeführte interventionelle Implantation einer Mitralklappenprothese möglich macht. Es ist ein relativ gerader Weg von der supraclaviculären Punktionsstelle, über den Venenwinkel, der Vena brachiocepahlica und der Vena cava superior zum rechten Vorhof und dem interatrialen Septum. Nach Durchdringen des Septums muss der Katheter nur leicht gebogen werden, um in den linken Vorhof zu gelangen. Hier ist wiederum nur eine leichte Biegung notwendig, um in den Mitralklappenapparat zu gelangen.

Im Vergleich zu einem transfemoralen Zugang ist die Entfernung von der Punktionsstelle zum Mitralklappenapparat mit nur ca. 15-20 cm sehr kurz.

Der Weg von der Punktion der Haut zum Venenwinkel ist sehr kurz. Dort hat die Vena brachiocephalica bereits einen großen Durchmesser und nach Zusammenfluss mit der Vena anonyma erweitet sie sich weiter zur Vena cava superior. Der Durchmesser der Vena brachiocephalica ist groß genug um Katheter mit einem Durchmesser von 40Fr. einzuführen.

Der supraclaviculäre Zugang erlaubt das Einführen eines großkalibrigen Mitralklappen-Katheters. Die Implantation kann unvergleichlich gut kontrolliert werden, da der Weg zum nativen Mitralklappenapparat kurz und vor allem auch gerade ist. Es ist kein starkes Verbiegen des Katheters notwendig, um in den nativen Mitralklappenapparat vorzudringen.

Um einen zentralen venösen Zugang zu erhalten wird üblicherweise die Vena subclavia punktiert. Die Vena subclavia liegt unter und hinter dem Schlüsselbein. Dieser Zugang wird üblicherweise genutzt, um einen großen Katheter einzuführen über den Flüssigkeiten und Medikamente zentral dem venösen Blut zugeführt werden können. Dieser Zugang wird jedoch auch genutzt, um Schrittmacherkabel an das Herz zu führen oder um eine Hämodialyse durchzuführen bis hin zum Anschluss einer Herz-Lungen-Maschine. Dies ist möglich, da die Vena subclavia einen großen Durchmesser hat und keine Venenklappen besitzt. Die Vena subclavia hat bei nahezu allen Patienten die gleiche Lage und ist so relativ sicher zu finden und zu punktieren.

Dennoch gibt es Situationen, in denen die Vena subclavia nicht für einen Zugang genutzt werden kann, z.B. nach Traumen, bei Verschluss oder bei liegenden Schrittmacherkabel.

Die vorliegende Erfindung erlaubt es aufgrund des hiermit vorgeschlagenen erfindungsgemäßen Positionierkatheter, erstmals vom supraclaviculären Zugang Gebrauch zu machen.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
Fig. 1: zeigt einen Führungskatheter in einer ersten Ausführungsform;
Fig. 1a: zeigt eine Vergrößerung eines Abschnitts aus Fig. 1;
Fig. 2: zeigt einen Hohlnadelkatheter zur Verwendung mit dem Führungskatheter;
Fig. 3: zeigt einen erfindungsgemäßen Positionierkatheter, in einer ersten Ausführungsform hiervon;
Fig. 3a: zeigt den distalen Endbereich und den distalen Teil des proximalen Endbereichs des Positionierkatheters der Fig. 3 in leicht vergrößerter Darstellung;
Fig. 4: zeigt einen Hämostasekatheter oder Ballonkatheter,;
Fig. 4a: zeigt den Ballonkatheter der Fig. 4 in einem nicht oder weniger expandierten Zustand;
Fig. 5, 5a: zeigen einen Mitralklappen-Katheter, in einer ersten Ausführungsform hiervon, in expandiertem Zustand der Mitralklappenprothese (Fig. 5) und in weniger expandiertem Zustand (Fig. 5a); und
Fig. 6a-6f: zeigen eine Ausführungsform eines Verfahrens zum Verwenden des Positionierkatheters.

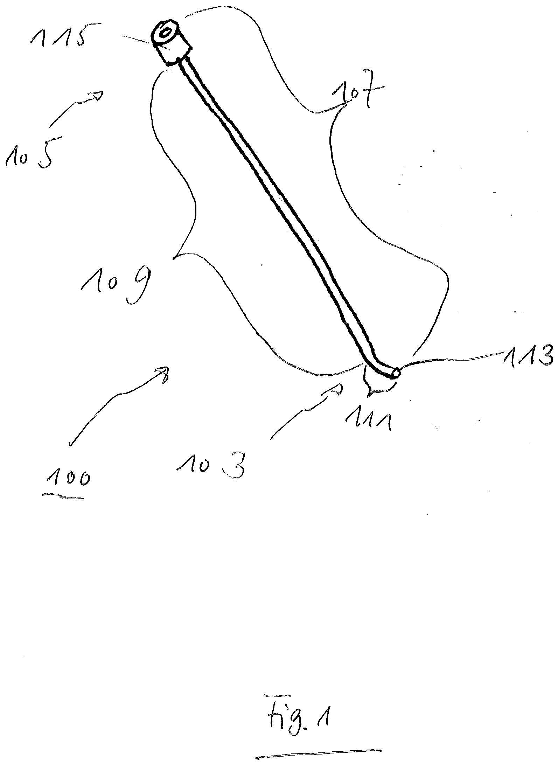

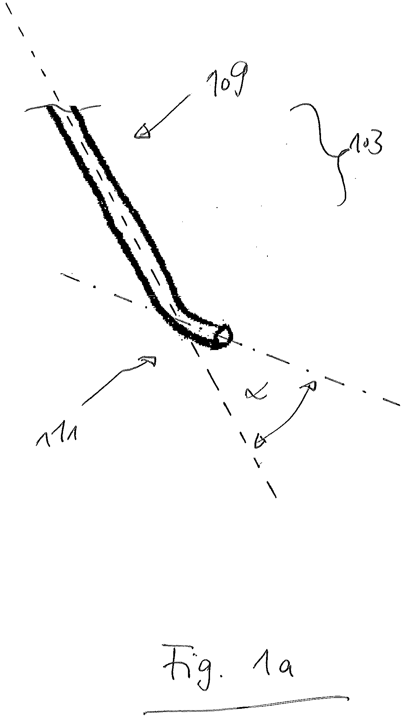

Fig. 1 zeigt einen Führungskatheter 100 in einer ersten Ausführungsform.

Der Führungskatheter 100 weist einen distalen Endbereich 103 und einen proximalen Endbereich 105 auf.

Von proximal nach distal erstreckt sich ein hohl ausgestalteten Führungsabschnitt 107. Er entspricht dem durchgehenden Teil des Führungskatheters 100, der das hohle Innere hat, also das Lumen, das sich vom distalen Ende bis zum proximalen Ende zieht.

Der Führungsabschnitt 107 einen geraden Abschnitt 109 und einen gekrümmten Abschnitt 111 auf oder besteht aus diesen beiden Abschnitten 109, 111.

Der gekrümmte Abschnitt 109 liegt im distalen Endbereich 103. In der Fig. 1 gezeigten exemplarischen Ausführungsform bildet der gekrümmte Abschnitt 111 das distale Ende des Führungsabschnitts 107. In anderen exemplarischen Ausführungsformen, welche hier nicht gezeigt sind, geht der gekrümmte Abschnitt 111 distal einer Krümmung, welche sich an den geraden Abschnitt 109 anschließt, erneut in einen weiteren, wenn gleich vorzugsweise sehr kurzen, geraden Abschnitt über.

Der gekrümmte Abschnitt 111 liegt distal zum geraden Abschnitt 109. Die Krümmung kann, wie in Fig. 1a , welche einem vergrößerten Abschnitt des distalen Endbereichs 103 aus Fig. 1 entspricht, gezeigt, rund 15° bis 90° betragen.

Das distale Ende des Führungsabschnitts 107 endet mit einer Öffnung 113, aus welcher ein im Führungsabschnitt 107 geführter Führungsdraht geführt werden kann.

Das proximale Ende des Katheters 100 weist ein optionales Ventil 115 auf. Das Ventil 115 ist wie der Führungsabschnitt 107 ebenfalls hohl. Sein Hohlraum fluchtet mit jenem des Führungsabschnitts 107, weshalb ein Führungsdraht sowohl durch den Führungsabschnitt als auch durch das Ventil 115 hindurch geschoben werden kann.

Fig. 1a zeigt den distalen Endbereich 103 des Führungskatheters 100 der Fig. 1 in vergrößerter Darstellung.

Die Längsachse des Hohlraums, welcher den geraden Abschnitt 109 durchzieht, ist als Strichlinie gezeichnet. Die Längsachse des Hohlraums, welcher den gekrümmten Abschnitt 111 durchzieht, ist als Strich-Punkt-Linie angedeutet. Fig. 1a zeigt, dass der gerade Abschnitt 109 und der gekrümmte Abschnitt 111 unter einem Winkel α zueinanderstehen. Der Winkel α ergibt die Krümmung, von welcher hier angenommen wird, dass ihr Entstehen ausschließlich der Form des gekrümmten Abschnitts 111 zuzurechnen ist. Es ist erfindungsgemäß sowohl vorgesehen, dass der gekrümmte Abschnitt 111 durchgehend gekrümmt ist, als auch dass der gekrümmte Abschnitt 111 neben der Krümmung auch einen geraden Abschnitt aufweist oder hiermit an der Öffnung 113 endet.

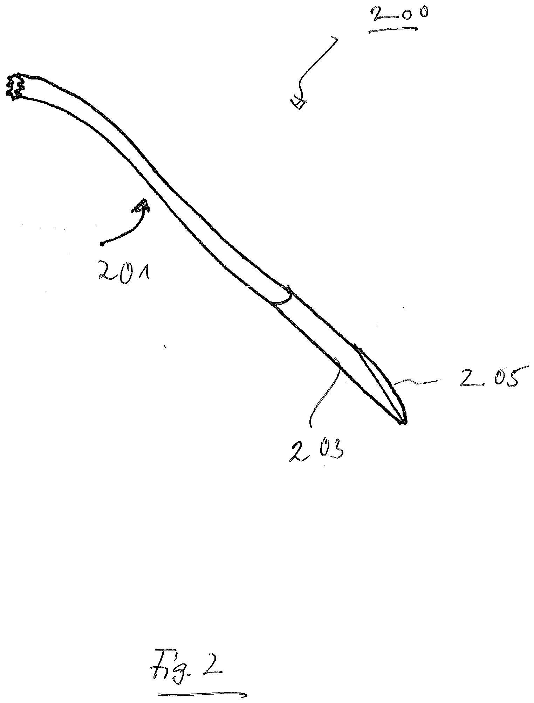

Fig. 2 zeigt einen Hohlnadelkatheter 200 zur Verwendung mit dem Führungskatheter 100.

Der Hohlnadelkatheter 200 weist wiederum einem hohlen Führungsabschnitt auf, durch welchen ein Führungsdraht in das Innere des Hohlnadelkatheters 200 eingebracht und relativ zu zu diesem bewegt werden kann.

Der hohle Führungsabschnitt ist wenigstens oder abschließend auf einen flexiblen Katheterabschnitt 201 und eine nicht flexible Hohlnadel 203 verteilt. Die flexible Hohlnadel 203 stellt das distale Ende des Hohlnadelkatheters 200 dar. Sie kann eine schräg verlaufende Schneide 205 aufweisen. "Schräg" bedeutet hier, dass die umlaufende Schneidkante der Schneide 205 keine runde, sondern eine ovale Fläche umschreibt.

Der Hohlnadelkatheter 200 kann dimensioniert sein, um im hohlen Führungsabschnitt 107 des Führungskatheters 100 aufgenommen werden zu können und um sowohl mit seiner Schneide 205 der Hohlnadel 203 als auch mit dem flexibler Katheterabschnitt 201 aus der Öffnung 113 des Führungskatheters 100 austreten zu können.

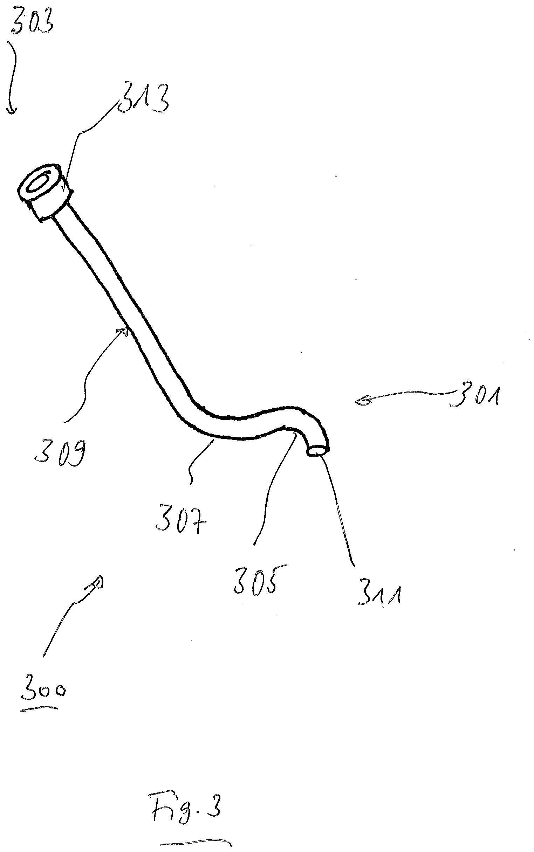

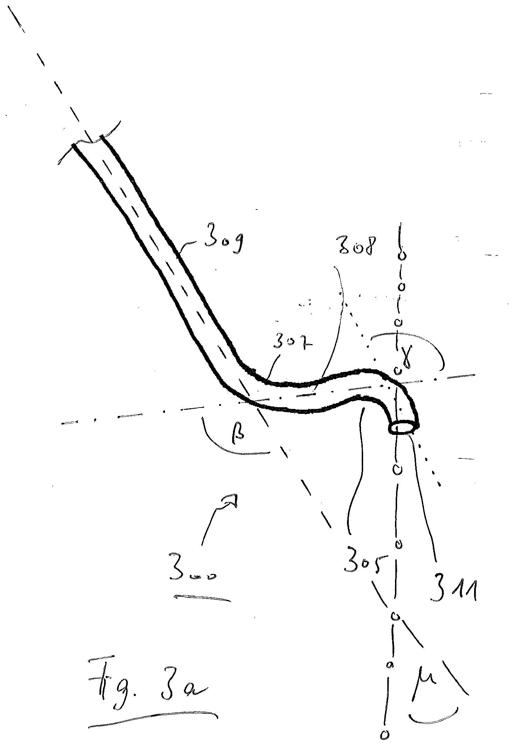

Fig. 3 zeigt einen erfindungsgemäßen Positionierkatheter 300, in einer ersten Ausführungsform hiervon.

Der Positionierkatheter 300 weist einen distalen Endbereich 301 und einen proximalen Endbereich 303 auf.

Anders als der Führungskatheter 100 der Fig. 1 weist der Positionierkatheter 300 wenigstens oder genau zwei Krümmungsabschnitte 305 und 307 auf, welche in einer gemeinsamen ersten Ebene (der Zeichenebene in Fig. 3) liegen und welche jeweils distal zu einem geraden Abschnitt 309 liegen.

Der Positionierkatheter 300 kann zusätzlich zu der vorstehend genannten Krümmung, welche in der ersten Ebene liegt, wenigstens oder genau eine Krümmung in einer zweiten Ebene aufweisen, wobei die zweite Ebene senkrecht auf der ersten Ebene steht. Die Krümmung in der zweiten Ebene kann dergestalt sein, dass die Öffnung 311 weiter über oder vor der Zeichenebene der Fig. 3 steht als wenigstes zwei andere Abschnitte des Positionierkatheters 300, die in der Zeichenebene liegen. Die Krümmung in der zweiten Ebene kann vorzugsweise zwischen - 45° und + 90° betragen.

Der distale Endbereich 301 weist eine Endöffnung oder Öffnung 311 eines Führungsabschnitts auf, welcher sich von der Öffnung 311 bis zu einem proximalen Ende des proximalen Endbereichs 303 erstreckt, ggf. bis ein auf das proximale Ende optional aufgesetztes Ventil 313.

Der in Fig. 3 in exemplarischer Ausgestaltung gezeigte Positionierkatheter 300 ist gekrümmt, um, wenn er durch das interatriale Septum 715 hindurch geschoben wird, mit seiner Öffnung 311 vor dem, oder im Bereich des, nativen Mitralklappenapparat(s) 719 zu stehen. Seine besondere Krümmung erlaubt es, ihn bei Zugang über die Vena brachiocephalica 711 oder Vena cave superior 713 auf einfache Weise bis in den linken Vorhof und vor die Mitralklappe 719 zu leiten. Dabei kann bereits der Positionierkatheter 300 das Mitralklappenimplantat 600 lösbar tragen. In einer alternativen Ausgestaltung ist vorgesehen, dass der Positionierkatheter 300 als Führungskatheter dient, durch dessen hohlen Führungsabschnitt hindurch ein weiterer Katheter, hierin als Mitralklappen-Katheter 500 oder als mitralklappentragender Katheter 500 bezeichnet, welcher das Mitralklappenimplantat 600 trägt, bis vor die native Mitralklappe 719 zu deren Ersatz geführt wird.

Der Positionierkatheter 300 kann derart steif ausgestaltet sein, dass sein Biegemodul steifer oder größer ist als das Biegemodul des Mitralklappen-Katheters 500, welcher das Mitralklappenimplantat 600 trägt, so dass der Positionierungskatheter 300 im Gebrauch, d. h., wenn das Mitralklappenimplantat 600 durch dessen Führungsabschnitt geführt wird, nicht an Form verliert.

In einigen erfindungsgemäßen Ausführungsformen ist jedoch das Gegenteil der Fall: Der Positionierkatheter 300 oder sein Führungsabschnitt haben dieselbe oder eine niedrigere Festigkeit oder dasselbe oder ein niedrigeres Biegemodul als das Mitralklappenimplantat 600.

Die vorstehend genannten Krümmungen oder Biegungen des Positionierkatheters sind derart geformt, dass die distale Öffnung 311 vorzugweise direkt oberhalb des nativen Mitralklappenapparats 719 oder im Mitralklappenapparat 719 zu liegen kommt.

Fig. 3a zeigt den distalen Endbereich 301 und den distalen Teil des proximalen Endbereichs 303 des Positionierkatheters 300 der Fig. 3 in leicht vergrößerter Darstellung.

Die Längsachse des Hohlraums, welcher den geraden Abschnitt 309 durchzieht, ist als Strichlinie gezeichnet. Die Längsachse des Hohlraums, welcher den ersten gekrümmten Abschnitt 305 durchzieht, ist als Punktlinie angedeutet. Die Längsachse des Hohlraums, welcher den zweiten gekrümmten Abschnitt 307 durchzieht, ist als Strich-Punkt-Linie angedeutet.

Fig. 3a zeigt, dass der gerade Abschnitt 309 und der zweite gekrümmte Abschnitt 307 unter einem Winkel β zueinanderstehen. Der Winkel β ergibt die erste Krümmung, von welcher hier angenommen wird, dass ihr Entstehen ausschließlich der Form des zweiten gekrümmten Abschnitts 307 zuzurechnen ist.

Fig. 3a zeigt, dass der zweite gekrümmte Abschnitt 307 und der erste gekrümmte Abschnitt 305 unter einem Winkel γ zueinanderstehen. Der Winkel γ ergibt die zweite Krümmung, von welcher hier angenommen wird, dass ihr Entstehen ausschließlich der Form des ersten gekrümmten Abschnitts 305 zuzurechnen ist.

Es ist erfindungsgemäß sowohl vorgesehen, dass der erste gekrümmte Abschnitt 305 durchgehend gekrümmt ist, als auch dass er neben der Krümmung auch einen geraden Abschnitt aufweist oder hiermit an der Öffnung 311 endet.

Der Winkel β kann zwischen 0° und 120°, vorzugsweise 20° bis 40° betragen.

Der Winkel γ kann zwischen 10° und 120°, vorzugsweise zwischen 90° und 120° betragen.

Ein Winkel µ welcher sich zwischen der Längsachse des Hohlraums, welcher den geraden Abschnitt 309 durchzieht (siehe die Strichlinie) und einer Austrittsrichtung für einen Austritt aus der Öffnung 311, gezeichnet mittels Strich-Kreis-Linie, beträgt in der Zeichenebene oder der Ebene der doppelten Krümmung vorteilhaft zwischen 0° (parallel) und 60°.

Ein zwischen der ersten Krümmung 305 und der zweiten Krümmung 307 (oder zwischen dem Schnittpunkt der Geraden, welche die Winkel β und γ ergeben) gelegener Abschnitt 308 ist erfindnungsgemäß zwischen 2 cm und 6 cm lang.

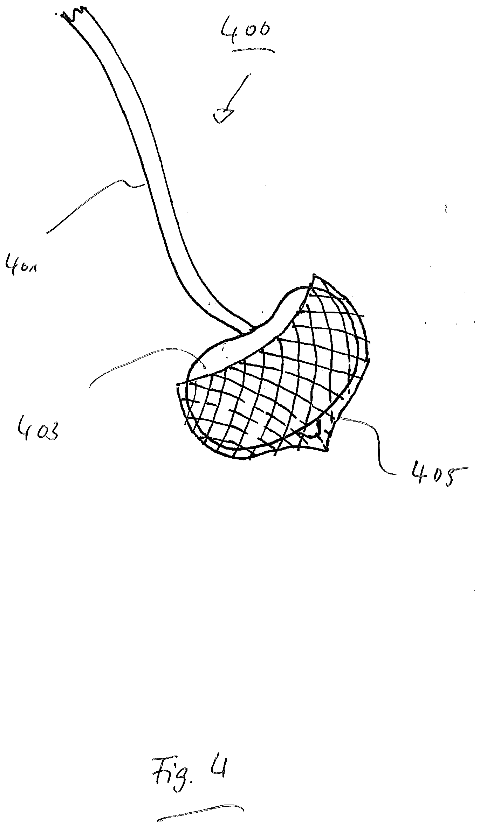

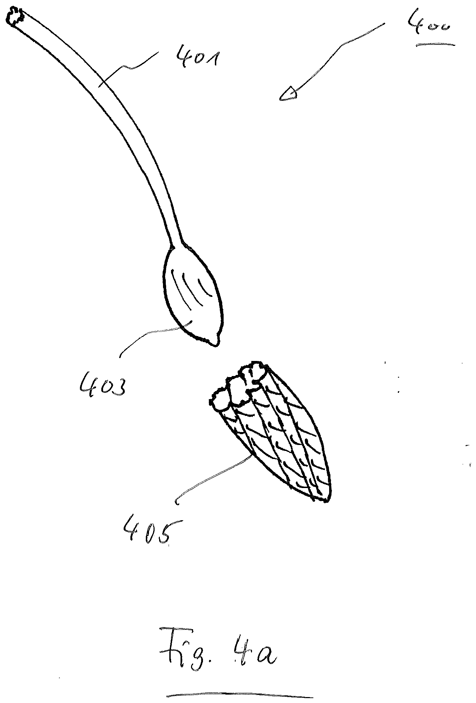

Fig. 4 zeigt einen Hämostasekatheter oder Ballonkatheter 400.

Der Ballonkatheter 400 hat einen Leitungsabschnitt 401 und einen hieran angesetzten und in Fluidkommunikation verbundenen Ballonabschnitt 403.

Der Ballonabschnitt 403 kann mittels Fluid, zumeist Kochsalzlösung, über den Leitungsabschnitt 401 befüllt und hierdurch expandiert oder aufgeweitet werden.

Optional weist der Ballonabschnitt 403 eine hämostatische, resorbierbare Hülle 405 auf, welche nach Einsatz des Ballonkatheters 400 in der Punktionswunde verbleiben und dort resorbiert werden kann.

Der Ballonabschnitt 403 ist in Fig. 4 expandiert gezeigt.

Fig. 4a zeigt den Ballonkatheter 400 der Fig. 4 in einem nicht oder weniger expandierten Zustand, in welchem er nach vollständigem oder teilweisem Ablassen des Fluids oder des im Balloninneren herrschenden Fluiddrucks aus der Punktionswunde entfernt werden kann. Die optionale Hülle 405 kann nach Deflation des Ballons oder Ballonabschnitts 403 in der Punktionswunde zurückbleiben. Sie kann dort resorbiert werden.

Fig. 5 und Fig. 5a zeigen einen exemplarischen erfindungsgemäßen Mitralklappen-Katheter 500, in einer ersten Ausführungsform hiervon.

Der Mitralklappen-Katheter 500 weist in einem distalen Endbereich 501 ein Mitralklappenimplantat 600 auf.

Das Mitralklappenimplantat 600 ist einmal im expandierten Zustand der Mitralklappenprothese (Fig. 5) gezeigt, einmal in weniger expandiertem oder gefaltetem Zustand ( Fig. 5a ) .

Das Mitralklappenimplantat 600 basiert auf einem faltbaren oder crimpbaren Stentkörper, welcher in Fig. 5 und Fig. 5a gezeigt ist, und Klappensegeln, welche in diesen Figuren nicht gezeigt sind.

Der Stentkörper ist vorzugsweise aus Nitinol oder einem anderen Shape-Memory-Material gefertig oder weist ein solches auf.

Der Stentkörper weist beispielhaft drei Längsverbinder oder Posts 605 auf, welche eine ringförmige distale Führungsstruktur 601 und eine ringförmige proximale Führungsstruktur 603 miteinander verbinden.

Durch oder um die ringförmige distale Führungsstruktur 601 und die ringförmige proximale Führungsstruktur 603 laufen Zügel oder Strings 617, welche auch in einem inneren Längslumen des Mitralklappen-Katheters 500 verlaufen. Die Strings 617 sind vorgesehen, um hiermit eine radial nach innen wirkenden Druck auf die ringförmige distale Führungsstruktur 601 und die ringförmige proximale Führungsstruktur 603 ausüben zu können mit dem Ziel, den Durchmesser dieser beiden Führungsstrukturen und damit auch den Durchmesser des Stentkörpers verringern zu können.

Für Details dieser beispielhaften der Mitralklappe wird auf die Ausführungen zur Herzklappe verwiesen, welche sich in der WO 2009/109348 A1 oder der WO 2015/014960 A1 finden, deren diesbezügliche Offenbarungen hiermit zum Gegenstand gemacht werden.

Losgelöst von allen mit Bezug auf Fig. 5 beschriebenen Merkmalen des Mitralklappenimplantats 600 kann dieses auf dem Mitralklappen-Katheter 500 oder auf dem Positonierkatheter 300 (z. B. auf dessen distalen Endabschnitt) derart vorgesehen sein, dass die Mitralklappensegel eine Durchströmung in Richtung distal nach proximal (bezogen auf die Anordnung des Mitralklappenimplantats 600 auf dem Katheter, in Fig. 5 von oben nach unten) erlaubt, in der umgekehrten Richtung jedoch unterbindet (in Fig. 5 also von unten nach oben). Das Mitralklappenimplantat 600 ist auf dem Katheter somit vorzugweise für eine Implantation angeordnet, bei welcher der Katheter vom linken Vorhof kommend auf die native Mitralklappe 719 hin geschoben wird, nicht vom linken Ventrikel her kommend.

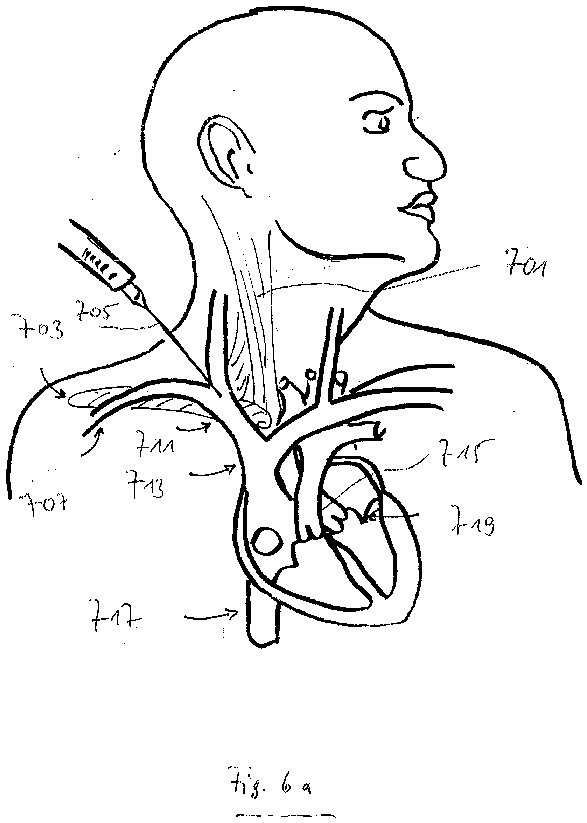

Die Fig. 6a bis 6f zeigen eine Ausführungsform eines Verfahrens zur Anwendung des Positionierkatheters an einem Patienten.

Fig. 6a zeigt die für die Implantierung des Mitralklappenimplants 600 erforderliche Punktierung. Im Beispiel der Fig. 6a wird der Winkel zwischen dem rechten Musculus sternocleidomastoideus 701 und der rechten Clavicula 703 identifiziert. Die Haut wird rund 1 cm neben dem Kopf des Musculus sternocleidomasteideus 701 und mit rund 1 cm Abstand zur Clavicula 703 punktiert. Die zur Punktion verwendete Nadel 705 wird in einem Winkel von 35-45° zur Körperachse und unter einem Winkel von ca. 15° zur Körperebene (vorzugweise gezielt auf die gegenüberliegende Brustwarze) eingeführt. Die Nadel 705 punktiert sodann den "Venenwinkel" der Vena subclavia 707 und der Vena jugularis interna 709, bei der Vereinigung zur Vena brachiocephalica 711.

In Fig. 6a sind ferner das interatriale Septum mit dem Bezugszeichen 715, die Vena cava inferior mit Bezugszeichen 717 und die Mitralklappe mit dem Bezugszeichen 719 bezeichnet.

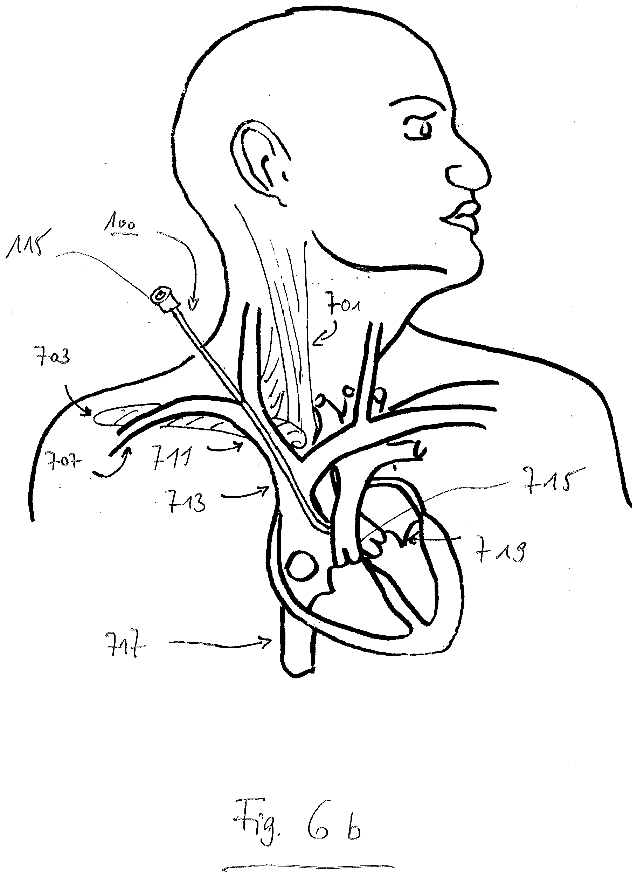

Fig. 6b zeigt die Vorbereitung der Punktion des interatrialen Septums 715. Hierzu wird nach mit Bezug zu Fig. 6a beschriebener supraclaviculärer Punktion der Vena brachiocephalica 711 wird der steife, am distalen Ende leicht gebogene erfindungsgemäße Führungskatheter 100 "over the wire" über die Vena brachiocephalica 711 und die Vena cava superior 713 mit der Spitze bzw. seiner Öffnung 113 zum interatrialen Septum 715 geführt.

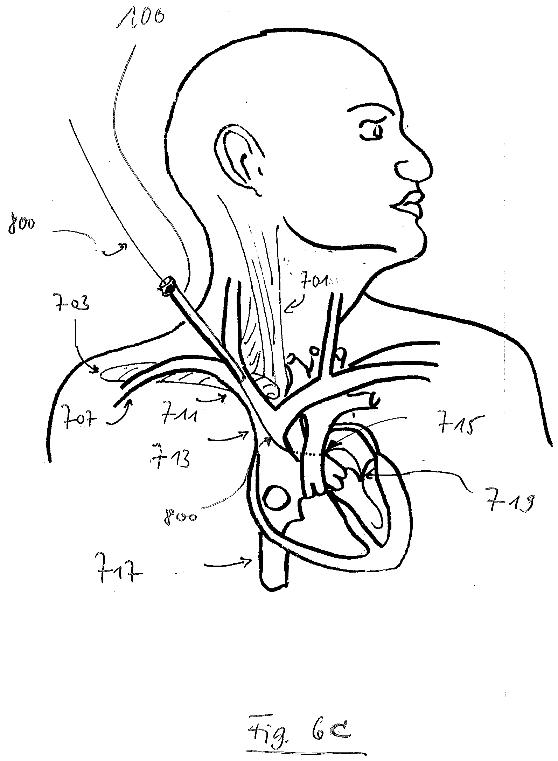

Fig. 6c zeigt den Zustand nach erfolgter Punktion des interatrialen Septums 715, zu welcher der Hohlnadelkatheter 200 in den hohlen Führungsabschnitt des steifen Führungskatheters 100 eingeführt wurde, und wobei mit der aus dessen Öffnung 113 austretenden Schneide 205 der Hohlnadel 203 das interatriale Septum 715 punktiert wurde.

Fig. 6c zeigt ferner, dass über den Hohlnadelkatheter 200 ein Führungsdraht 800 in den linken Vorhof und durch die mittels Punktion mit der Hohlnadel 203 geschaffene Öffnung im interatrialen Septum 715 weiter in den linken Ventrikel geschoben wurde. Der Führungskatheter 100 ist bereits zur Hälfte wieder aus dem Körper des Patienten herausgezogen.

Eine Dilation der mittels Hohlnadel 203 im interatrialen Septum 715 geschaffenen Öffnung kann mittels eines Dilatators oder Ballons geweitet werden.

Durch die Öffnung im interatrialen Septum 715 kann im nächsten Schritt der distale Endabschnitte des Positionierkatheters 300 "over the wire" in die Vena brachiocephalica und die Vena cava superior transseptal in den linken Vorhof eingeführt werden.

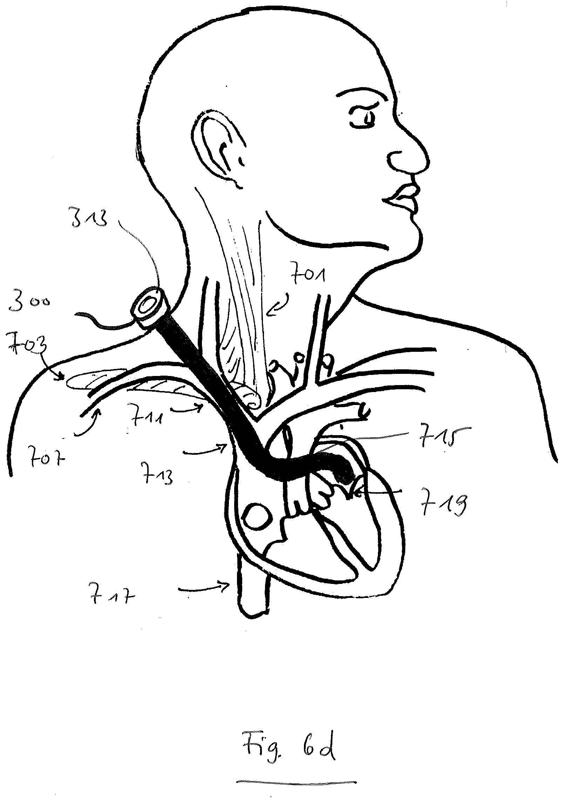

Fig. 6d zeigt diesen Zustand. Fig. 6d zeigt, dass die Biegung des erfindungsgemäßen Positionierkatheters 300 es erlaubt, dessen offenes Ende genau über oder auch in dem nativen Mitralklappenapparat zu positionieren. Nach Positionierung des Positionierkatheters 300 wird die Mitralklappenprothese 600 entlang des Führungsabschnitts durch das Innere des Positionierkatheters 300 vorgeschoben und im nativen Mitralklappenapparat 719 verankert.

Ebenso kann über den liegenden, d. h. eingeführten Positionierkatheter 300 eine Mitralklappenreparatur durchgeführt werden.

Fig. 6e zeigt einen Zustand des Patienten nach Implantation des Mitralklappenimplantats 600.

Dem Patienten wird über den vorhandenen Zugang ein hämostatischer Ballonkatheter 400 in das subkutane Gewebe bis direkt vor der Punktionsstelle der Vena brachiocephalica 711 vorgeschoben. Dies geschieht vorteilhaft über einen Führungsdraht, welcher in Fig. 6e nicht gezeigt ist. Der in Fig. 6e gezeigte Zustand entspricht damit dem Zustand nach Einbringen des Ballonkatheters 400 und nach Entfernen des Führungsdrahts, sofern ein solcher verwendet wurde. Der Ballonabschnitt 403 wird im subkutanen Gewebe befüllt und damit geweitet und führt zu einer sofortigen Blutstillung.

Sobald eine ausreichende Blutgerinnung und Blutstillung erfolgt ist, was einige Minuten dauern kann, wird der Ballon entlüftet und entfernt. Die hämostatische Hülle verbleibt am Ort und gewährleistet so eine weitere Blutstillung. Fig. 6f zeigt diesen Zustand. Die hämostatische Hülle ist vorzugsweise resorbierbar.

### Bezugszeichenliste

100: Führungskatheter
103: distaler Endbereich
105: proximaler Endbereich
107: hohler Führungsabschnitt
109: gerader Abschnitt
111: gekrümmter Abschnitt
113: Öffnung oder Austrittsöffnung
115: Ventil
200: Hohlnadelkatheter
201: flexibler Katheterabschnitt
203: Hohlnadel
205: Schneide
300: Positionierkatheter
301: distaler Endbereich
303: proximaler Endbereich
305: erster Krümmungsabschnitt
307: zweiter Krümmungsabschnitt
308: Abschnitt
309: gerader Abschnitt
311: Öffnung
313: Ventil
400: Ballonkatheter
401: Leitungsabschnitt
403: Ballonabschnitt
405: Hülle
500: Mitralklappen-Katheter
501: distaler Endbereich
600: Mitralklappenimplantat, künstliche Mitralklappe, Mitralklappenprothese
601: ringförmige distale Führungsstruktur
603: ringförmige proximale Führungsstruktur
605: Längsverbinder oder Posts
617: Zügel oder Strings
701: Musculus sternocleidomastoideus
703: Clavicula
705: Nadel
707: Vena subclavia
709: Vena jugularis interna
711: Vena brachiocephalica
713: Vena cava superior
715: interatriales Septum
717: Vena cava inferior
719: Mitralklappe, Mitralklappenapparat
800: Führungsdraht

## Patentansprüche

1. Medizinischer Positionierkatheter (300) zur Verwendung beim Implantieren eines Mitralklappenimplantats (600) oder zum Reparieren eines Herzdefekts oder einer Mitralklappe (719), wobei der Positionierkatheter (300) aufweist:
einen distalen Endbereich (301), welcher ein distales Ende des Positionierkatheters (300) umfasst, und einen proximalen Endbereich (303), welcher ein proximales Ende des Positionierkatheters (300) umfasst;
einen hohl ausgestalteten Führungsabschnitt;
wobei der Führungsabschnitt einen geraden Abschnitt (309) und wenigstens einen ersten Krümmungsabschnitt (305) und einen zweiten Krümmungsabschnitt (307) aufweist;
wobei der erste Krümmungsabschnitt (305) und der zweite Krümmungsabschnitt (307) im distalen Endbereich (301) liegen oder wobei der erste Krümmungsabschnitt (305) oder der zweite Krümmungsabschnitt (307) das distale Ende bildet;
wobei der erste Krümmungsabschnitt (305) und der zweite Krümmungsabschnitt (307) distal zum geraden Abschnitt (309) angeordnet sind,
wobei der erste Krümmungsabschnitt (305) eine Biegung oder Krümmung des Führungsabschnitts um 10 bis 120°, bevorzugt um 90 bis 120° bewirkt,
**dadurch gekennzeichnet, dass** der zweite Krümmungsabschnitt (307) eine Biegung oder Krümmung des Führungsabschnitts um 20 bis 40° bewirkt und
dass zwischen dem ersten Krümmungsabschnitt (305) und dem zweiten Krümmungsabschnitt (307) ein 2 - 6 cm langer Abschnitt (308) angeordnet ist.

2. Positionierkatheter (300) nach Anspruch 1, welcher ferner aufweist:
ein Ventil (313), das den geraden Abschnitt (309) nach proximal abschließt.

3. Positionierkatheter (300) nach einem der Ansprüche 1 bis 2, wobei der erste Krümmungsabschnitt (305) und der zweite Krümmungsabschnitt (307) gegensinnig gekrümmt sind.

4. Positionierkatheter (300) nach einem der Ansprüche 1 bis 3, wobei der Positionierkatheter (300) oder sein Führungsabschnitt maximal 80 cm, bevorzugt maximal 60 cm, besonders bevorzugt maximal 45 cm lang sind.

5. Positionierkatheter (300) nach einem der Ansprüche 1 bis 4, wobei der Positionierkatheter (300) oder sein Führungsabschnitt nicht-flexibel, nicht-elastisch oder steif sind.

6. Positionierkatheter (300) nach einem der Ansprüche 1 bis 5, wobei der Positionierkatheter (300), sein Führungsabschnitt oder der erste Krümmungsabschnitt (305) und der zweite Krümmungsabschnitt (307) eine shape-memory-Funktion oder -form haben oder aus einem shape-memory-Material bestehen oder ein solches aufweisen.

7. Positionierkatheter (300) nach einem der Ansprüche 1 bis 6, wobei die Durchströmungsrichtung des Mitralklappenimplantats (600), wenn dieses auf dem Positionierkatheter (300) vorliegt, vom proximalen Endbereich (303) des Positionskatheters (300) zum distalen Endbereich (301) verläuft.

## Claims

1. A medical positioning catheter (300) for use when implanting a mitral valve implant (600) or for repairing a heart defect or a mitral valve (719), wherein the positioning catheter (300) has:
a distal end region (301) which comprises a distal end of the positioning catheter (300), and a proximal end region (303) which comprises a proximal end of the positioning catheter (300);
a hollow guide section;
wherein the guide section has a straight section (309) and at least a first curved section (305) and a second curved section (307);
wherein the first curved section (305) and the second curved section (307) lie in the distal end region (301) or wherein the first curved section (305) or the second curved section (307) form the distal end;
wherein the first curved section (305) and the second curved section (307) are disposed distally to the straight section (309);
wherein the first curved section (305) causes the guide section to be deflected or curved by 10° to 120°, preferably by 90° to 120°,
**characterized in that** the second curved section (307) causes the guide section to be deflected or curved by 20° to 40°, and
**in that** a 2 to 6 cm long section (308) is disposed between the first curved section (305) and the second curved section (307).

2. The positioning catheter (300) as claimed in claim 1, which further has:
a valve (313) which closes the straight section (309) proximally.

3. The positioning catheter (300) as claimed in claim 1 or claim 2, wherein the first curved section (305) and the second curved section (307) are curved in opposite directions.

4. The positioning catheter (300) as claimed in one of claims 1 to 3, wherein the positioning catheter (300) or its guide section are a maximum of 80 cm long, preferably a maximum of 60 cm long, particularly preferably a maximum of 45 cm long.

5. The positioning catheter (300) as claimed in one of claims 1 to 4, wherein the positioning catheter (300) or its guide section is non-flexible, non-elastic or rigid.

6. The positioning catheter (300) as claimed in one of claims 1 to 5, wherein the positioning catheter (300), its guide section or the first curved section (305) and the second curved section (307) have a shape memory function or form or consist of a shape memory material or the like.

7. The positioning catheter (300) as claimed in one of claims 1 to 6, wherein the perfusion direction of the mitral valve implant (600), when it is on the positioning catheter (300), runs from the proximal end region (303) of the positioning catheter (300) to the distal end region (301).

## Revendications

1. Cathéter de positionnement (300) médical, destiné à être utilisé lors de l'implantation d'un implant de valvule mitrale (600) ou à réparer une anomalie cardiaque ou une valvule mitrale (719), le cathéter de positionnement (300) comportant :
une zone d'extrémité distale (301), laquelle comprend une extrémité distale du cathéter de positionnement (300), et une zone d'extrémité proximale (303), laquelle comprend une extrémité proximale du cathéter de positionnement (300) ;
un tronçon de guidage conçu sous forme creuse ;
le tronçon de guidage comportant un tronçon droit (309) et au moins un premier tronçon incurvé (305) et un deuxième tronçon incurvé (307) ;
le premier tronçon incurvé (305) et le deuxième tronçon incurvé (307) se situant dans la zone d'extrémité distale (301) ou le premier tronçon incurvé (305) ou le deuxième tronçon incurvé (307) formant l'extrémité distale ;
le premier tronçon incurvé (305) et le deuxième tronçon incurvé (307) étant placés de manière distale par rapport au tronçon droit (309),
le premier tronçon incurvé (305) provoquant une flexion ou une courbure du tronçon de guidage d'au moins 10 à 120°, de préférence, de 90 à 120°,
**caractérisé en ce que** le deuxième tronçon incurvé (307) provoque une flexion ou une courbure du tronçon de guidage de 20 à 40 ° et
**en ce qu'**entre le premier tronçon incurvé (305) et le deuxième tronçon incurvé (307) est placé un tronçon (308) long de 2 à 6 cm.

2. Cathéter de positionnement (300) selon la revendication 1, lequel comporte par ailleurs :
une soupape (313), qui termine le tronçon droit (309) en direction proximale.

3. Cathéter de positionnement (300) selon l'une quelconque des revendications 1 à 2, le premier tronçon incurvé (305) et le deuxième tronçon incurvé (307) étant incurvés à contresens.

4. Cathéter de positionnement (300) selon l'une quelconque des revendications 1 à 3, le cathéter de positionnement (300) ou son tronçon de guidage étant d'une longueur maximale de 80 cm, de préférence maximale de 60 cm, de manière particulièrement préférentielle, maximale de 45 cm.

5. Cathéter de positionnement (300) selon l'une quelconque des revendications 1 à 4, le cathéter de positionnement (300) ou son tronçon de guidage étant non flexible, non élastique ou rigide

6. Cathéter de positionnement (300) selon l'une quelconque des revendications 1 à 5, le cathéter de positionnement (300), son tronçon de guidage ou le premier tronçon incurvé (305) et le deuxième tronçon incurvé (307) présentant une fonction ou une configuration à mémoire de forme ou étant constitué d'une matière à mémoire de forme ou comportant une telle matière.

7. Cathéter de positionnement (300) selon l'une quelconque des revendications 1 à 6, la direction de circulation à travers l'implant de valvule mitrale (600), lorsque celui-ci est présent sur le cathéter de positionnement (300) s'écoulant de la zone d'extrémité proximale (303) du cathéter de positionnement (300) vers la zone d'extrémité distale (301).
